# EUROPÄISCHE PATENTANMELDUNG

(11) **EP 1 970 434 A1**
(43) Veröffentlichungstag der Anmeldung: **17.09.2008**
(21) Anmeldenummer: 08152440.7
(22) Anmeldetag: 07.03.2008
(51) Int. Cl.: C12M 1/107, C12M 1/04

(54) **Verfahren zum Abbau von Schwefelwasserstoff mittels Zuführen von Sauerstoff**

(30) Priorität: 15.03.2007 DE 102007013190
(71) Anmelder: MT-Energie GmbH & Co. KG, 27404 Rockstedt (DE)
(72) Erfinder: Martens, Christoph, 27404 Rockstedt (DE)
(74) Vertreter: von Ahsen, Erwin-Detlef

(57) **Zusammenfassung**

Die Erfindung betrifft ein Verfahren zum Abbau von Schwefelwasserstoff in einem Biogasfermenter (10) zum Erzeugen von Biogas aus Biomasse, welche als Gärsubstrat dem Biogasfermenter (10) zugeführt wird. Es ist bekannt, Luft in einen Biogasfermenter (10) zu führen. Als ein Bestandteil der Luft dient Sauerstoff dazu, schwefelwasserstoffabbauende Bakterien zu versorgen. Mit der Luft wird dem Biogas ein störender Anteil an Stickstoff zugeführt. Zur Vermeidung dieses Nachteils wird erfindungsgemäß vorgeschlagen, einem Gasraum (26) des Biogasfermenters (10) ein Reaktionsgas mit gegenüber Luft erhöhtem Sauerstoffgehalt zuzuführen.

## Beschreibung

Die Erfindung betrifft ein Verfahren zum Abbau von Schwefelwasserstoff in einem Biogasfermenter zum Erzeugen von Biogas aus Biomasse, welche als Gärsubstrat dem Biogasfermenter zugeführt wird.

Ein solches Verfahren ist aus der DE 10 2005 041 798 A1 bekannt.

Bei der Produktion von Biogas entsteht Schwefelwasserstoff. Schwefel ist für die Ernährung von Organismen ein unentbehrliches Element und gelangt daher zwangsläufig mit dem Substrat in Form von Sulfaten und Sulfiden in den Gärreaktor von Biogasanlagen. Aufgrund der anaeroben biologischen Abbauvorgänge entsteht durch die teilweise Umwandlung der gebundenen Schwefelverbindungen Schwefelwasserstoff. Besonders bei der Vergärung eiweißreicher Substanzen wird im Gärbehälter eine erhebliche Menge an gasförmigen Schwefelwasserstoff gebildet.

Bei der Verbrennung des Biogases entsteht aus dem Schwefelwasserstoff das giftige Schwefeldioxid. Des Weiteren führen Schwefelwasserstoff und Schwefeldioxid an beispielsweise Armaturen oder Motorbauteilen zu Beschädigungen aufgrund von Korrosion und können zu einer beschleunigten Motorölversäuerung beitragen. Dies verursacht hohe Reparatur- und Wartungskosten und begründet daher die nachhaltige Notwendigkeit einer effektiven und preisgünstigen Biogasentschwefelung.

Die Biogasentschwefelung wird zukünftig noch an Bedeutung gewinnen. So muss das Biogas vor einem Einsatz in Brennstoffzellen vom Schwefelwasserstoff befreit werden, da sich dieser direkt auf wichtige Bestandteile der Zelle auswirkt. Des Weiteren muss der Methananteil des Biogases erhöht werden, um dieses in die bestehenden Erdgasleitungsnetze einspeisen zu können. Hierzu ist es insbesondere auch erforderlich, den Methananteil im Biogas auf das Niveau von Erdgas zu erhöhen, da sonst der Brennwert des Mischgases absinkt. Deshalb ist es notwendig, das Biogas von Kohlendioxid beispielsweise über eine Gaswäsche oder Filterung zu befreien. Dieses erfolgt beispielsweise über eine Aminwäsche, welche ein nahezu vollständig von Schwefelwasserstoff befreites Biogas erfordert. Das so auf Erdgasqualität aufbereitete Biogas lässt sich nicht nur in das Erdgasleitungsnetz einspeisen, sondern auch zum Betanken von Erdgasfahrzeugen an entsprechenden Tankstellen verwenden.

Es ist bekannt, Luft in einen Biogasfermenter zu führen. Als ein Bestandteil der Luft dient Sauerstoff dazu, schwefelwasserstoffabbauende Bakterien zu versorgen. Über die Bakterien wird der Schwefelwasserstoff entweder in reinen Schwefel und Wasser oder in schwefelige Säure umgewandelt.

Bei diesem bekannten Entschwefelungsverfahren ist von Nachteil, dass durch das Einblasen von Luft nicht nur der für die Umwandlung des Schwefelwasserstoffes benötigte Sauerstoff mit einem Anteil von etwa 21 % in den Gasraum geführt wird. Mit einem Anteil von etwa 79 % wird durch die Zuführung von Luft auch nicht erforderlicher Stickstoff zugeführt. Er wird Bestandteil des Biogases und mindert den Brennwert und damit die Qualität des Biogases. Zwar ist in der DE 10 2005 041 798 A1 in Absatz [0005] die Rede davon, dass der Schwefelwasserstoff in elementaren Schwefel umgewandelt wird, wenn dem Fermenter im Gasraum Sauerstoff zugeführt wird. Letztlich ist hiermit der Sauerstoff aber nur als Bestandteil der Luft als das durch die Schwefelwasserstoff abbauenden Mikroorganismen umgesetzte Gas gemeint. Da der Sauerstoff selbst im anaeroben Gärprozess im Fermenter störend, nämlich sogar für die das Methangas bildenden Mikroorganismen toxisch ist, ist die Fachwelt stets bestrebt gewesen, so wenig Sauerstoff wie möglich in den Fermenter einzubringen. Dieses belegt auch die DE 199 38 853 A1. Diese Schrift betrifft ein Verfahren, bei dem zunächst ein anaerober Gärprozess und sodann ein aerober Kompostierungsprozess durchgeführt wird. Hierzu stehen entweder zwei gesonderte Behälter zur Verfügung, oder das Verfahren wird in ein und demselben Behälter in zeitlich aufeinanderfolgender Zeitfolge durchgeführt. Der Anlage ist eine Lufttrenneinrichtung zugeordnet, durch welche die Luft in ihre Hauptbestandteile Stickstoff und Sauerstoff aufgeteilt wird. Der so gewonnene Stickstoff wird dem Prozess während des anaeroben Prozesses zugeführt, während der gewonnene Sauerstoff während des aeroben Kompostierungsprozesses zugeführt wird. Grund für die Trennung ist auch hier, dass der Sauerstoff während des anaeroben Gärprozesses störend ist.

Weiterhin ist aus der DE 10 2004 035 997 A1 eine Biogasanlage bekannt, wobei es sich hierbei konkret um einen Holzvergaser, in welchem ein Pyrolyseprozess stattfindet, handelt. Bei einem konkreten Ausführungsbeispiel in diesem Stand der Technik wird aufbereitete Luft mit einem erhöhten Anteil von Sauerstoff zur Pyrolyse bereitgestellt. Dieses dient aber nicht dem Entschwefelungsprozess, sondern rein der Pyrolyse. Die Entschwefelung erfolgt, wie an anderer Stelle dieser Schrift genannt, durch Lufteinblasung.

Ferner ist durch die AT 007985 U1 eine Vorrichtung zum Entfernen von schwefelhaltigen Verbindungen von Biogas bekannt. Die Schwefelverbindungen werden hier in einem dem eigentlichen Biogasreaktor nachfolgenden Absorber mittels Sauerstoff abgebaut. Der dem eigentlichen Biogasfermenter nachfolgende Absorber stellt eine zusätzliche Anlagenkomponente mit zusätzlichen Kosten dar.

Schließlich zeigt die DE 197 44 653 A1 eine Biogasanlage, bei der ebenfalls eine Entschwefelung durch Sauerstoffzufuhr erfolgt. Das in dem eigentlichen Biogasreaktor gewonnene Biogas wird in einem gesonderten Gasdom zwischengespeichert, in welchen der Sauerstoff zugeführt wird. Hierdurch ist eine gewisse räumliche Trennung zwischen den das Methangas bildenden Mikroorganismen im Gärsubstrat und den Sauerstoff umsetzenden, Schwefelwasserstoff abbauenden Mikroorganismen im Gasdom gegeben. Letztlich ist aber auch in dieser Schrift der Sauerstoff nur als Bestandteil der Luft angesprochen.

Das der Erfindung zugrundeliegende Problem ist es, den Stickstoffanteil des Biogases präventiv zu verhindern und damit die Qualität des über einen Biogasfermenter produzierten Biogases nachhaltig zu verbessern.

Zur Lösung des Problems ist das erfindungsgemäße Verfahren dadurch gekennzeichnet, dass in einem Gasraum des Biogasfermenters ein Reaktionsgas mit gegenüber Luft erhöhtem Sauerstoffgehalt zugeführt wird.

Durch die erfindungsgemäße Maßnahme wird auf überraschend einfache Weise der Schwefelwasserstoffabbau im Biogas begünstigt, ohne unnötig viel Stickstoff mit in das Biogas einzutragen. Dieses ist vor allem deshalb überraschend, da der Gärprozess zum Erzeugen des Biogases anaerob abläuft; erfindungsgemäß aber dem Biogas ein Gas mit erhöhtem Sauerstoffgehalt direkt in den Fermenter zugeführt wird. Ferner ist im Vergleich zur Verwendung von Luft eine geringere Gasvolumenmenge nötig, die in den Gärraum zugeführt werden muss. Aufgrund des höheren Sauerstoffgehaltes reicht ein geringeres Gasvolumen aus, um die gleiche Menge Schwefelwasserstoff umzuwandeln und die zugeführte Menge an Stickstoff zu reduzieren, wodurch auch der Anteil des Stickstoffs am Biogas bereits präventiv vermindert wird. Der Methananteil des Biogases erhöht sich, wodurch die Qualität des Biogases gesteigert wird. Zudem kann der erhöhte Sauerstoffanteil im zugeführten Gas zu einer wesentlichen Optimierung der Entschwefelung führen, wenn an ungünstig zu erreichenden Stellen unter Umständen nur ein Teil der zugeführten Gasmenge für die entschwefelnden Mikroorganismen zur Verfügung steht.

Nach einer Weiterbildung der Erfindung wird an einem Auslass für das Biogas aus dem Biogasfermenter die Menge von Schwefelwasserstoff und/oder Stickstoff und/oder Sauerstoff im Biogas gemessen und die Menge des zugeführten Reaktionsgases in Abhängigkeit der gemessenen Werte geregelt. Hierdurch wird sichergestellt, dass immer nur die Menge an Reaktionsgas dem Biogasfermenter zugeführt wird, welche für den Schwefelwasserstoffabbau erforderlich ist. Ist der Anteil an Schwefelwasserstoff zu hoch, wird die Reaktionsgaszufuhr erhöht, ist der Sauerstoffgehalt zu hoch, wird die Reaktionsgaszufuhr reduziert. Hierdurch ist sichergestellt, dass der in den Biogasfermenter eingebrachte Sauerstoff möglichst vollständig durch die Schwefelwasserstoff abbauenden Mikroorganismen verbraucht wird und nicht in das Gärsubstrat gelangen kann, wo er für die Methangas bildenden Mikroorganismen toxisch wäre.

Die obengenannte Wirkung wird weiter dadurch verbessert, dass das Reaktionsgas mittels Zuführleitungen möglichst direkt an Bewuchsflächen für die Schwefelwasserstoff abbauenden Mikroorganismen zugeführt wird. Der im Reaktionsgas zugeführte Sauerstoff kann so direkt von diesen Mikroorganismen aufgenommen werden und nicht in das Gärsubstrat gelangen.

Nach einer Weiterbildung der Erfindung kann das Gas mit einem erhöhten Sauerstoffgehalt durch eine Anreicherung von Luft mit Sauerstoff erzeugt werden. Insbesondere kann das Gas erst unmittelbar vor dem Zuführen durch Anreichern der Luft mit Sauerstoff erzeugt werden. Das zuzuführende Gas kann also direkt an der Anlage produziert werden. Eine Produktion des Gases an anderer Stelle und der unter Umständen aufwendige und kostenintensive Transport zur Anlage und Lagerung sind somit nicht nötig.

Des Weiteren ist es von Vorteil, wenn technisch reiner Sauerstoff zugeführt wird. Hierdurch kann die injizierte Gasmenge bei der biologischen Luftentschwefelung um bis zu 79 % reduziert werden. Die Effektivität der Luftentschwefelung wird somit noch einmal gesteigert. Der störende Stickstoffanteil wird gar nicht erst in den Gasraum eingebracht.

Entsprechend einer vorteilhaften Ausführungsform des erfindungsgemäßen Verfahrens wird dem Biogasfermenter eine Sauerstoffzufuhr zum Zuführen eines Gases mit gegenüber Luft erhöhtem Sauerstoffgehalt zugeordnet.

Nachfolgend wird die Erfindung anhand einer beispielhaften Ausführungsform mittels einer Zeichnung erläutert. Es zeigt:
- Fig. 1: ein erstes Ausführungsbeispiel einer Vorrichtung mit den Erfindungsmerkmalen in einer schematischen Seitenansicht,
- Fig. 2: ein weiteres Ausführungsbeispiel einer Vorrichtung mit den Erfindungsmerkmalen in einer schematischen Seitenansicht.

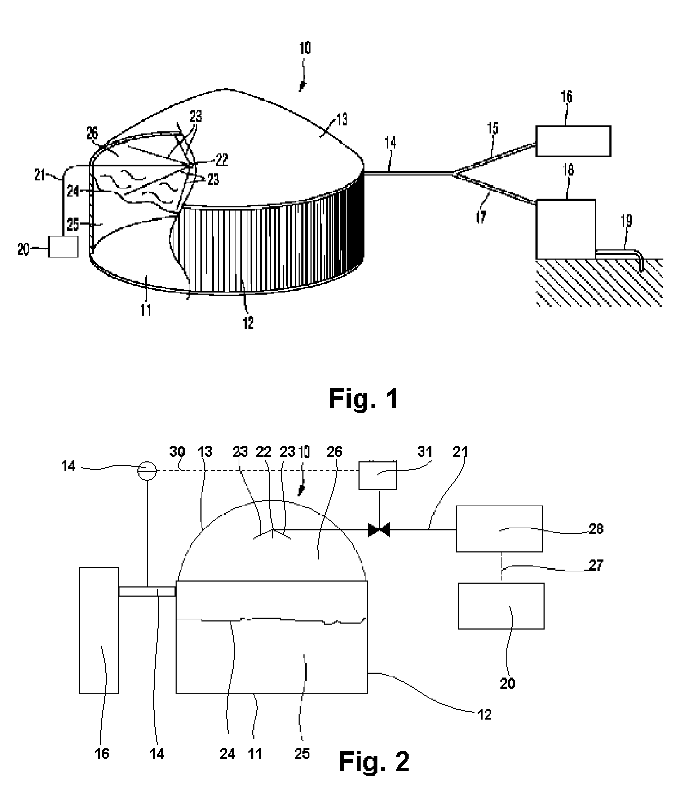

Die Figur 1 zeigt eine schematische Ansicht eines Biogasfermenters 10. Der Biogasfermenter 10 weist eine kreisförmige Bodenplatte 11 und eine umlaufende Behälterwand 12 auf. Zur Bedeckung des Biogasfermeters 10 dient eine Tragluftfolienabdeckung 13. In dem Biogasfermenter 10 befindet sich ein Gärsubstrat 25 aus welchem durch anaeorbes Vergären ein Biogas entsteht. Dieses gelangt durch eine Gärsubstratsoberfläche 24 in einen Gasraum 26 oberhalb des Gärsbustrates 25 im Biogasfermenter 10. Das Biogas wird über eine Gasleitung 14 aus dem Gasraum 26 des Biogasfermeters 10 abgeführt. Über eine sich von der Gasleitung 14 abzweigende Zweigleitung 15 kann das Biogas direkt einem Verbraucher 16, beispielsweise einem Blockheizkraftwerk, zugeführt werden. Über eine weitere sich von der Gasleitung 14 abzweigende Zweigleitung 17 kann das Biogas zusätzlich oder alternativ einer Gasreinigung 18 zugeführt werden.

Über die Gasreinigung 18 wird der Methananteil im Biogas auf das Niveau von Erdgas erhöht. Hierzu kann die Gasreinigung 18 das Biogas über eine Gaswäsche oder Filterung vom Kohlendioxid befreien. Das durch die Gasreinigung 18 vorbereitete Biogas wird anschließend dem Erdgasleitungsnetz 19 zugeführt. Die Gasreinigung 18 kann beispielsweise über eine Aminwäsche erfolgen. Diese erfordert jedoch ein nahezu vollständig von Schwefelwasserstoff (H₂S), welcher beim Gärprozess immer entsteht, befreites Biogas.

Eine Möglichkeit, Schwefelwasserstoff abzubauen, ist mittels entsprechender Bakterien, welche dem Schwefelwasserstoff unter Aufnahme von Sauerstoff in Schwefel und Wasser (2H₂S + O₂ → 2S + 2H₂O) oder schwefelige Säure (2H₂S + 3O₂ → 2H₂SO₃) umwandeln. Hierzu ist es nötig, die schwefelwasserstoffabbauenden Bakterien im Biogasfermenter 10 mit Sauerstoff zu versorgen.

An sich herrschen im Gasraum 26 oberhalb der Gärsubstratoberfläche 24 ideale Lebensbedingungen für die schwefelwasserstoffabbauenden Bakterien mit Ausnahme des fehlenden Sauerstoffs. Sauerstoff an dieser Stelle könnte aber in das mittels eines Rührwerkes (nicht dargestellt) ständig in Bewegung gehaltene Gärsubstrat 25 gelangen und dort den anaeroben Gärprozess stören. Tatsächlich ist dieses aber nicht der Fall.

Dem Biogasfermenter 10, nämlich in dessen Gasraum 26, wird ein Gas mit gegenüber Luft erhöhtem Sauerstoffgehalt, konkret technisch reiner Sauerstoff, zugeführt. Zu diesem Zweck ist dem Biogasfermenter 10 ein Sauerstoffgenerator 20 zugeordnet, welcher technisch reinen Sauerstoff aus der Umgebungsluft erzeugt. Dieser Sauerstoff wird also direkt an der Anlage produziert und über eine Zuleitung 21 zu einem Verteiler 22 geführt. Der Verteiler 22 verteilt das Gas über Verteilungsleitungen 23 gleichmäßig und über der Gärsubstratsoberfläche 24. Konkret sind die Verteilungsleitungen 23 so ausgebildet, dass sie den Sauerstoff direkt an Besiedelungsflächen für die Schwefelwasserstoff abbauenden Mikroorganismen zuleiten, so dass der zugeführte Sauerstoff möglichst direkt von diesen Organismen aufgenommen werden kann.

Bei dem in Fig. 2 gezeigten Ausführungsbeispiel wird der im Sauerstoffgenerator 20 generierte Sauerstoff zunächst über eine Leitung 27 einem Speicher 28 zugeleitet und hier zwischengespeichert. Hierdurch steht immer eine ausreichende Menge an Sauerstoff zur Verfügung.

Ferner wird an einer Messtelle 29 an der Gasleitung 14, durch welche das Biogas aus dem Biogasfermenter 10 abgeleitet wird, die Zusammensetzung des Biogases gemessen. Konkret wird die Biogas enthaltene Menge an Schwefelwasserstoff, Stickstoff und/oder Sauerstoff bestimmt und über eine Datenleitung 30 einer Regelung 31 zur Verfügung gestellt. Diese regelt in Abhängigkeit der gemessenen Werte über ein Ventil 32 die dem Gasraum 36 zugeleitete Menge an Sauerstoff. Ist der Schwefelwasserstoffgehalt in der Gasleitung 14 zu hoch, also der Schwefelwasserstoff nicht hinreichend vollständig genug abgebaut, wird die Menge an Sauerstoff erhöht. Ist hingegen kein oder kaum Schwefelwasserstoff enthalten, dafür aber die Sauerstoffmenge zu hoch, wird die Menge an zugeführtem Sauerstoff verringert. Wird, wie oben angedeutet, zusätzlich auch die Stickstoffmenge im Biogas in der Gasleitung 14 bestimmt, kann diese für eine Regelung der Trennung der Luft in Sauerstoff und Stickstoff im Sauerstoffgenerator 20 geregelt werden, falls für bestimmte Anwendungen doch eine bestimmte Menge an Stickstoff im Biogas gewünscht sein sollte.

Durch die Zuführung des über dem Sauerstoffgenerator 20 bereitgestellten Sauerstoffes kann das in den Biogasfermenter 10 eingebrachte Gasvolumen reduziert werden, da im Vergleich zur Verwendung von Luft das Gas einen erhöhten Sauerstoffanteil besitzt. Gleichzeitig kann der nicht erforderliche Stickstoffanteil, insbesondere bei der Verwendung von technisch reinem Sauerstoff, auf ein Minimum reduziert werden.

### Bezugszeichenliste:

- 10: Biogasfermenter
- 11: Bodenplatte
- 12: Behälterwand
- 13: Tragluftfolienabdeckung
- 14: Gasleitung
- 15: Zweigleitung
- 16: Verbraucher
- 17: Zweigleitung
- 18: Gasreinigung
- 19: Erdgasleitungnetz
- 20: Sauerstoffgenerator
- 21: Zuleitung
- 22: Verteiler
- 23: Verteilerleitungen
- 24: Gärsubstratsoberfläche
- 25: Gärsubstrat
- 26: Gasraum
- 27: Leitung
- 28: Speicher
- 29: Meßstelle
- 30: Datenleitung
- 31: Regelung
- 32: Ventil

## Patentansprüche

1. Verfahren zum Abbau von Schwefelwasserstoff in einem Biogasfermenter (10) zum Erzeugen von Biogas aus Biomasse, welche als Gärsubstrat dem Biogasfermenter (10) zugeführt wird, **dadurch gekennzeichnet, dass** in einem Gasraum (26) des Biogasfermenters (10) ein Reaktionsgas mit gegenüber Luft erhöhtem Sauerstoffgehalt zugeführt wird.

2. Verfahren nach Anspruch 1, **dadurch gekennzeichnet, dass** an einem Auslass (14) für das Biogas aus dem Biogasfermenter (10) die Menge von Schwefelwasserstoff und/oder Stickstoff und/oder Sauerstoff im Biogas gemessen und die Menge des zugeführten Reaktionsgases in Abhängigkeit der gemessenen Werte geregelt wird.

3. Verfahren nach Anspruch 1 oder 2, **dadurch gekennzeichnet, dass** das Reaktionsgas mittels Zuführleitungen (23) an Besiedelungsflächen für Schwefelwasserstoff abbauende Mikroorganismen zugeführt wird.

4. Verfahren nach einem der Ansprüche 1 bis 3, **dadurch gekennzeichnet, dass** das Gas mit erhöhtem Sauerstoffgehalt durch Anreicherung von Luft erzeugt wird.

5. Verfahren nach Anspruch 4, **dadurch gekennzeichnet, dass** das Gas mit erhöhtem Sauerstoffgehalt unmittelbar vor der Zuführung in den Biogasfermenter (10) erzeugt wird.

6. Verfahren nach einem der vorhergehenden Ansprüche, **dadurch gekennzeichnet, dass** technisch reiner Sauerstoff in den Biogasfermenter (10) zugeführt wird.
